# EUROPEAN PATENT APPLICATION

(11) **EP 0 786 269 A1**
(43) Date of publication of application: **30.07.1997**
(21) Application number: 97100932.9
(22) Date of filing: 22.01.1997
(51) Int. Cl.: A61N 1/375, H01R 4/28, H01R 13/10

(54) **Fixation device**

(30) Priority: 29.01.1996 SE 9600311
(71) Applicant: Pacesetter AB, 171 95 Solna (SE)
(72) Inventor: Sivard, Ake, S-171 58 Solna (SE)

(57) **Abstract**

The present invention relates to a fixation device (2) for affixing an elongate contact pin (8) on an electrode lead and is intended for connection to a medical implant, such as a heart stimulator. The device contains an elongate connector part (4) with an opening (6) at one end into which the contact pin (8) can be inserted. The connector part (4) has a fixation part (36) which, when acted on by the contact pin (8), can move in the connector part's longitudinal direction between a contact pin-fixation position and a contact pin-release position.

## Description

The present invention relates to a fixation device according to the preamble to patent claim 1.

The connecting parts of pacemakers often consist of a molded-on section containing an electrode connection terminal, the molded-on section being made of transparent epoxy plastic mounted on the exterior of the pacemaker enclosure. The electrode connection terminals in the molded-on section are in electrical contact with the pacemaker circuits in the enclosure via a number of connection pins. The proximal end of the electrode cable has a contact pin is secured inside the molded-on section e.g. with screws. In a description of the prior art, the American patent document US-5086773 supplies examples of such solutions. The device described in US-5086773 is an example of a way the contact pin can be affixed without the need for screws or tools. The locking device described in US-5086773 contains a coil spring arranged in a connection socket for affixing the contact pin. The internal diameter of the coil spring is somewhat smaller than the external diameter of the contact pin. When the contact pin is rotated part of one turn in the coil spring's uncoiling direction at the same time as the contact pin is pushed into the connection socket, the spring expands enough to allow the contact pin to be pushed into the spring. When the contact pin is released, the spring strives to return to its normal position, thereby gripping the contact pin and affixing the contact pin on the electrode cable. One problem with this prior art device is that the spring could be broken if the contact pin, secured by the locking device, is mistakenly rotated in the spring's coiling direction, i.e. opposite to the spring's uncoiling direction.

Another device for affixing an electrode cable in a connection means on a heart stimulator is described in EP-A2-0590756. The fixation device consists of a spring plate, molded inside the connection means, with opposed resilient, clamping tongues designed to grip the cable's contact pin to prevent the end of the electrode cable from being pulled out of the connection means, the retention force of the clamp increasing when a withdrawal force is exerted on the electrode cable. The grip of the clamping tongues on the contact pin on the end of the cable loosens when pressure is applied to opposed, projecting lateral tabs on the spring plate. The problem with this prior art device is that the contact pin could be released by mistake if pressure is applied to the lateral tabs.

The object of the present invention is to propose an improved fixation device which does not require any tool to achieve fixation and which cannot be released by the application of clamping on the connection means.

This object is achieved with a fixation device, as initially described above, and with the features set forth in the characterizing part of the independent claim.

With the invention, fixation of a contact pin to a connector part is therefore achieved by the incorporation of a fixation part, inside the connector part, which is able to move in the connector part's longitudinal direction, when force is applied to the contact pin, between a contact pin-fixation position and a contact pin-release position.

The fixation part is spring-loaded and pushes against the contact pin, securing it when the fixation part is in its fixation position. When a force is applied to the contact pin and the pin is pushed in the contact pin's longitudinal direction towards the connector part, the fixation part's fixation parts are released and the contact pin can then be withdrawn from the connector part.

When the contact is to be secured in the connector part, the contact pin is pushed with a predetermined force a predetermined distance into the connector part in the contact pin's longitudinal direction, the pin then being secured, when released, as the fixation part assumes its fixation position.

So the invention relates to the fixation of a contact pin for an electrode lead intended for connection to a medical implant, for example a pacemaker or defibrillator. A general requirement in the design of these implants is for them to be as small as possible. The latest generation of pacemakers only weighs 14 grams. A standard type of contact pin for connecting an electrode lead to a pacemaker has an external diameter of 1.62 mm. This will give some idea of the size of the fixation device.

As noted above, contemporary pacemakers have a molded-on epoxy section into which the electrode lead is connected.

According to the invention connection will instead be made directly into the pacemaker enclosure through a "black hole" without fixation screws. The fixation device is inside the pacemaker enclosure. This solution means that the molded-on section currently employed will not be needed to achieve lead connection.

According to a first preferred embodiment of the invention, the fixation device comprises a connector part containing a cylindrical guide body, pressing against a main spring acting on the fixation part and movingly arranged in the connector part, with rib-like, longitudinal projections on the surface of the guide body. The connector part further contains an inner and outer tubular guide arranged to interact with the tips of the longitudinal projections when there is a shift between the contact pin-fixation position and contact pin-releasing position.

According to a second preferred embodiment of the invention, the fixation device comprises a connector part containing an upper and a lower blocking part with a first and a second blocking tab on each blocking part to interact with the fixation part when there is a shift between the contact pin-fixation position and the contact pin-releasing position.

The invention will now be described in greater detail, referring to the FIGURES in which
FIG. 1 shows a first preferred embodiment of the fixation device according to the invention;
FIGS. 2a-2d shows a part, in different positions, to elucidate fixation with the first preferred embodiment of the fixation device according to the invention;
FIG. 3 shows an alternative version of the first preferred embodiment of the fixation device according to the invention;
FIGS. 4a-4d shows a second preferred embodiment of the fixation device according to the invention in different stages of fixation;
FIG. 5 shows a cross-section of the second preferred embodiment of the fixation device according to the invention.

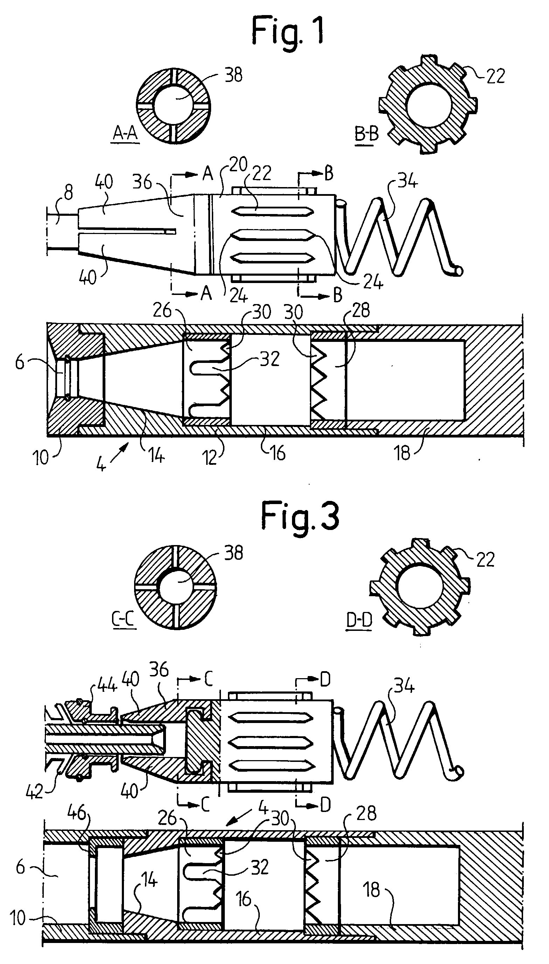

FIG. 1 shows the invention according to a first preferred embodiment. To facilitate description of the invention, the FIG. shows the fixation device in two parts and two cross-sections (A-A and B-B).

The fixation device 2 contains an elongate, preferably tubular, connector part 4 with an opening 6 through which a contact pin 8 can be inserted for fixation to the connector part 4. The connector part comprises an opening part 10, arranged inside of which is a tubular middle part 12, which consists of two parts, a first section 14, nearest the opening part, which forms a space in the connector part 4 in the shape of a truncated cone with the smaller diameter pointing towards the opening 6, and a second section 16, which forms a cylindrical space connected to the conical space. The cylindrical space formed by the second section of the middle part 12 changes into an additional cylindrical space formed by an inner part 18 which caps the connector part 4.

A cylindrical, movingly arranged guide body 20 is inside the cylindrical space formed by the second section 16. The length of the guide body 20 is approximately the same as the length of the second section. The surface of the guide body has a number, preferably 6-8, longitudinal, rib-like projections 22 running parallel to the guide body's 20 longitudinal direction and extending along part of the guide body's length. These projections preferably have a rectangular profile. The tips 24 of the projections have one steeply angled side and one shallowly angled side on either side of the tip.

An outer tubular guide 26 is arranged inside the middle part 12, where the first section 14 changes into the second section 16, and encircles the outer part of the guide body 20. An inner tubular guide 28, encircling the inner part of the guide body 20, is mounted at the point at which the second section 16 changes to the space formed by the inner part 18.

The inner guide 28 has a number of identical notches 30 arranged on the part facing the opening 6. These notches have an steeply angled side and a shallowly angled side arranged to mesh with the corresponding steeply angled and shallowly angled sides of the tips 24 of the projections on the guide body 20.

The outer guide 26 has a number of notches 30,32 arranged on the part facing away from the opening 6. Every other notch is deeper, and every other notch is shallower. The shallow notches 30 have a steeply angled side and a shallowly angled side arranged to mesh with the corresponding steeply angled and shallowly angled sides of the projections on the guide body 20.

The inner guide and outer guide respectively have the same number of notches whose number is equal to twice the number of projections on the guide body.

As a result of their interaction with the inner and outer guides, the projections on the guide body limit the guide body's movement in the connector part's longitudinal direction.

A tensioned main spring 34 is arranged between the guide body 20 and the cap on the inner part 18 and presses the guide body 20 towards the opening 6. As a result of the spring force, the projections 22 on the guide body 20 push against the notches 30,32 in the outer guide (26).

Because of the spring force, the guide body 20 pushes against the fixation part 36 arranged in the conical space formed by the first section 14 of the middle part 12. The fixation part 36 has a concentricity located opening 38 for admitting the contact pin.

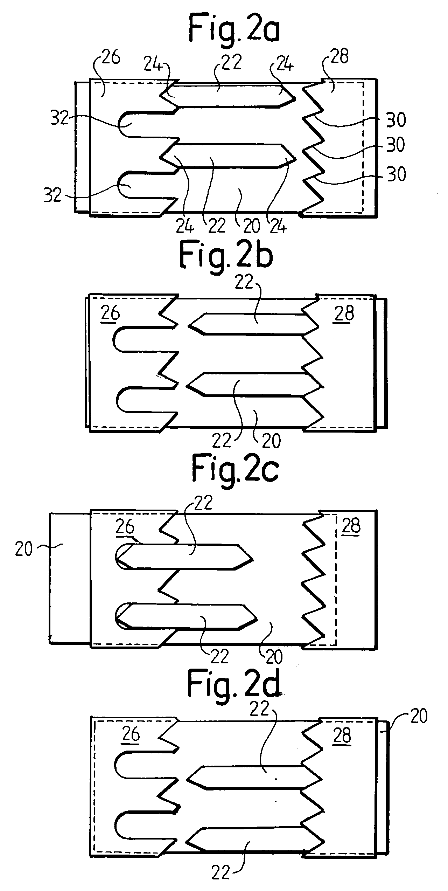

Referring to FIGS. 2a-2d, the function of the fixation device according to the first preferred embodiment will now be explained.

FIG. 2a shows the guide body 20 and the inner and outer guides 28,26. As a result of the spring force, the tips 24 of the projections, with their steeply angled and shallowly angled sides, push against the shallow notches 30 in the outer guide 26. When a contact pin (not shown) is inserted into the connector part, it causes the guide body 20 to be pushed back towards the inner guide 28 until the shallowly angled sides of the projections meet the corresponding shallowly angled sides on the inner guide 28, forcing the control body 20 to rotate (downwards in the FIG.) until the tips of the projections bottom in the inner guide's notches (see FIG. 2b). When the force, exerted by the contact pin along the connector part against the spring terminates, the main spring 34 pushes the guide body 20 back so the projections are pushed down, because the guide body has rotated, into the deep notches 32 in the outer guide 26. When the projections are seated in the deep notches, the fixation part 36 is pushed towards the opening part, and the fixation part, as a result of the opening part's conical shape, is pushed in towards the connector part's center axis, thereby exerting a fixation force on the contact pin which is accordingly secured in the connector part.

When the contact pin is to be detached (see FIG. 2c), force is applied to the contact pin against the connector part, causing the guide body 20 to push back against the inner guide 28 until the shallowly angled sides of the projections meets the corresponding shallowly angled sides of the inner guide 28, forcing the guide body 20 to rotate (downwards in the FIG) until the tips of the projections bottom in the notches of the inner guide (see FIG. 2d). When the force exerted by the contact pin along the connector part against the main spring 34 terminates, the main spring 34 pushes the guide body 20 back so the projections are pushed down, because the guide body 20 has rotated, down into the shallow notches 30 in the outer guide 26.

When the projections are seated in the shallow notches in the outer guide 30, the fixation part no longer presses against the opening part and is accordingly unable to exert any fixation force on the contact pin. The contact pin can therefore be detached.

As noted above, the fixation part has an opening 38 to admit a contact pin, this opening 38 continuing a distance into the guide body 20. The fixation part comprises a plurality, e.g. four, of preferably wedge-shaped clamping sections 40 designed, e.g. by the use of longitudinal slots, to make the clamping sections sufficiently radially mobile to clamp and hold the contact pin when acted upon by the spring force.

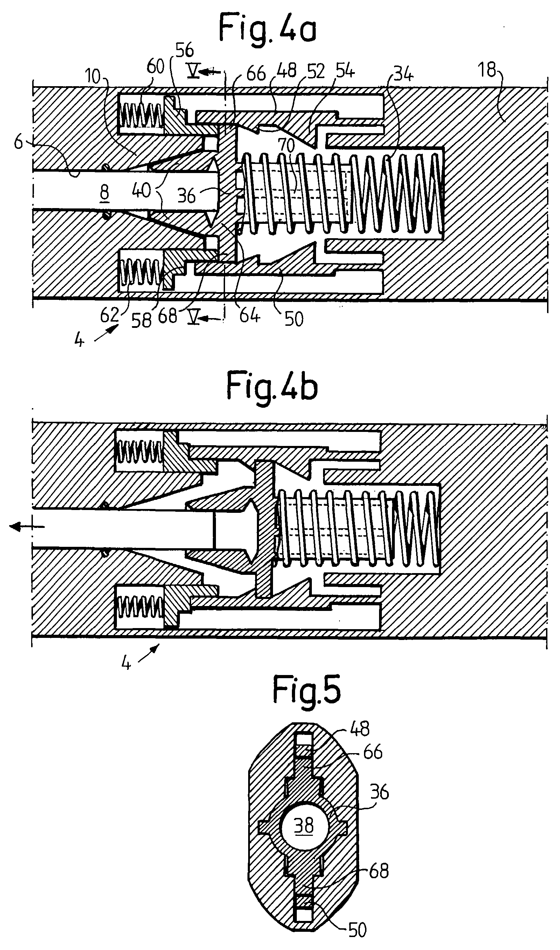

FIG. 3 shows an alternative design for the first preferred embodiment of the fixation device according to the invention. According to the above-described embodiment, the contact pin actuates the guide body by pushing directly against it. In order to achieve this, the contact pin must have a minimum length. According to an alternative to this first embodiment, an isolation flange 42, which is arranged near the contact pin, is instead made to push against an outer collar 44 which, in turn, presses against the fixation part 36. Fixation and release of the contact pin are performed in the same way as was described above. The advantage of this alternative version of the first preferred embodiment is that it works irrespective of the length of the contact pin, since it is the isolation flange 42, not the contact pin, which acts on the fixation part and guide body. A stop 46 is arranged near the outer collar 44 to keep the collar from being pulled off. Two cross-sectional views (C-C, D-D) of the fixation device are shown in conjunction with FIG. 3.

In the first preferred embodiment, electrical contact with the contact pin can be established in different ways, e.g. via the opening part 14 and the clamping sections on the fixation part 40, to the contact pin or via the inner part 18, main spring 34, guide body 20 and the fixation part's clamping sections 40 to the contact pin 8.

FIG. 4a-4d show a side view of the fixation device according to the invention according to a second preferred embodiment in different stages of contact pin-fixation. In the description of the second embodiment, the parts which functionally tally with the corresponding parts in the first preferred embodiment have been assigned the same names and reference designations.

Thus, this second embodiment of the fixation device comprises a connector part 4 which can have an oval cross-section. See FIG. 5 which shows a section at line A-A in FIG. 4a. In a similar manner as in the first embodiment, the connector part 4 has an opening part 10 with an opening 6 through which a contact pin 8, which is to be secured, is inserted into the connector part 4. The opening 6 changes to a conical space which, in turn, changes into an essentially cylindrical space formed by an inner part. A first and second blocking part 48,50 run from this inner part along the longitudinal axis of the connector part towards the opening. Two cavities are formed by the blocking parts 48,50 and the outer wall of the connector part above and below the blocking parts. When actuated, the blocking parts are bent into these cavities. A first and a second wedge-shaped blocking stud 52,54, facing the center axis of the connector part, are arranged on the blocking parts. One blocking stud has one sloping edge facing the opening and one radially aligned edge facing away from the opening. The second stud is about twice as high as the first stud. The first stud is located nearest the opening.

The blocking parts 48,50 interact with two gauge blocks, a first and a second gauge block 56,58 arranged on the blocking parts towards the opening. The gauge blocks are acted upon by a first spring and second spring 60,62 in a direction away from the opening and parallel to the longitudinal axis of the connector part towards the blocking parts 48,50.

The connector part has a fixation part 36, with an opening 38 for the contact pin, movingly arranged in the connector part's longitudinal direction. The fixation part consists of a wedge-shaped, clamping section 40 nearest the opening, a guide 64 with a first and a second part 66,68 and a tubular capping section 70.

A main spring 34 is arranged in the tensioned state in the inner part 18 between the rear wall of the inner part, partially encircling the tubular section 70 of the fixation part and pressing against the fixation part's guide 64.

FIG. 4a shows the contact pin secured by the fixation device according to the invention. The main spring 34 presses against the guide 64 and pushes the clamping section 40 into the conical space. The guide's first and second parts 66,68 push against the first and second gauge blocks 56,58 respectively which are pushed back, since the first and second spring 60,62 jointly exert much less force than the main spring 34. The clamping section 40 is so flexible that it applies a radial fixation force to the contact pin, when the pin is inserted into the conical space, thereby affixing the pin in the connector part.

When the contact pin is detached, a force, directed into the connector part, is applied to the contact pin, the fixation part 36 then being displaced, and the guide's first and second parts 66,68 cause the first and second blocking parts 48,50, when contacting the first blocking studs 52, to bend outward in the cavities until the guide passes the stud 52, and the blocking parts rebound. The fixation part is now held in place by the first blocking studs, and the clamping force exerted by the clamping sections ceases, making it possible for the contact pin to be withdrawn (see FIG. 4b).

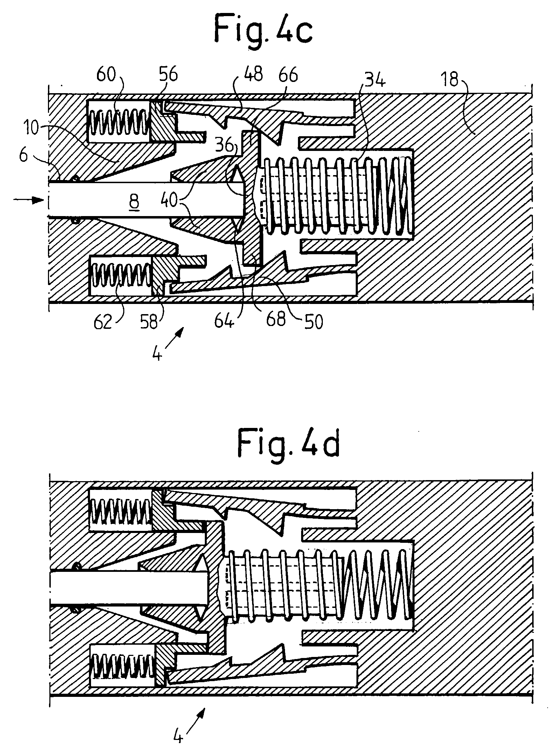

In fixation of the contact pin, the starting point is the one shown in FIG. 4b. The contact pin 8 is inserted into the connector part (see FIG. 4c), pressing the fixation part 36 against the main spring 34. The guide's first and second parts 66,68 cause the blocking parts 48,50, via the second blocking studs 54, to bend outward, and the gauge blocks 56,58, in a position near the connector part's outer wall, can, due to the force exerted by the first and second spring 60,62, move into the connector part and hold the blocking parts 48,50 in the bent-out position, as they rest on recesses in the gauge blocks 56,58. Since the blocking parts 48,50 are in this bent-out position, the guide 64, due to the force exerted by the main spring 34, can move towards the opening, and the guide's first and second sections 66,68 can pass the first blocking studs (see FIG. 4d). The guide's first and second parts 66,68 move the gauge blocks 56,58 towards the opening, parallel to the connector part's longitudinal axis, and the blocking parts return to their normal position (see FIG. 4a). This secures the contact pin as described above.

Electrical contact with the contact pin in the second preferred embodiment can be established in different ways, analogous to the method used for the first embodiment, e.g. via the opening part 10 and the clamping sections of the fixation part 40 to the contact pin 8 or via the internal part 18 of the connector part, the main spring 34 and the clamping sections of the fixation part 40 to the contact pin 80.

### Reference designations

- 2: Fixation device
- 4: Connector part
- 6: Opening
- 10: Opening part
- 12: Middle part
- 14: First section
- 16: Second section
- 18: Inner part
- 20: Guide body
- 22: Rib-like projections
- 24: Projection tip
- 26: Outer tubular guide
- 28: Inner tubular guide
- 30: Shallow notch
- 32: Deep notch
- 34: Main spring
- 36: Fixation part
- 38: Opening in fixation part
- 40: Clamping sections on the fixation part
- 42: Isolation flange
- 44: Outer part
- 46: Stop
- 48: First blocking part
- 50: Second blocking part
- 52: First blocking stud
- 54: Second blocking stud
- 56: First gauge block
- 58: Second gauge block
- 60: First spring
- 62: Second spring
- 64: Guide
- 66: First part of guide
- 68: Second part of guide
- 70: Tubular section

## Claims

1. A fixation device (2) for affixing an elongate contact pin (8) for an electrode lead intended for connection to a medical implant, e.g. a heart stimulator, said device comprising an elongate connector part (4) with an opening (6) at one end through which the contact pin (8) can be inserted into the connector part (4), and a fixation part (36) is arranged in the connector part, characterized in that the fixation part (36), when acted on by the contact pin (8) in the connector part's longitudinal direction, is able to move between a contact pin- fixation position and a contact pin-release position.

2. A fixation device according to claim 1, characterized in that the fixation part (36) is arranged to switch between the contact pin (36) fixation position and the contact pin-release position when the contact pin is pushed a predetermined distance into the connector part (4) and released.

3. A fixation device according to claim 1 or 2, characterized in that the fixation part (36) contains at least two inward clamping sections (40) between which the contact pin (8) passes during its insertion into the connector part (4), said sections pushing against the contact pin, when the fixation part is in the contact pin-fixation position, as a result of the pressure exerted by a tensioned main spring (36), arranged in the connector part, which exerts a force on the fixation part in the connector part's longitudinal direction towards the opening (6).

4. A fixation device according to claim 3, characterized in that the connector part contains blocking means (22,26,52,66,68) which, when the fixation part is in the contact pin-release position, locks the said clamping sections in a position without fixation contact with the contact pin.

5. A fixation device according to claim 4, characterized in that the connector part contains a cylindrical guide body (20) which pushes against the main spring (34) and which is movingly arranged in the connector part to act on the fixation part, the control body having rib-like longitudinal projections on is surface, and the guide body is arranged between two axially separate inner and outer tubular gauge blocks (26,28), the guide body and the gauge blocks being arranged so the fixation part, through the interaction of the projections and gauge blocks, can be locked in the contact pin-fixation position or the contact pin-release position.

6. A fixation device according to claim 5, characterized in that the surface of the guide body (20) has a number, preferably 6-8, of longitudinal, rib-like projections 22 parallel to the guide body's 20 longitudinal axis and extending along part of the guide body's (20) length, these projections having a rectangular cross-section and a tip (24), with one steeply angled side and one shallowly angled side, at either end of the projections, the said tips and steeply angled sides and shallowly angled sides arranged to mesh with notches (30,32) in the outer (26) and inner (29) tubular guides.

7. A fixation device according to claim 4, characterized in that the connector part (4) contains two opposed blocking parts (48,50) for interaction with the fixation part (36) in order to lock the fixation part in the contact pin-release position against the force exerted by the main spring (34) or release the fixation part from this position in order to move it to the contact pin-fixation position.

8. A fixation device according to claim 7, characterized in that each blocking part contains a first (52) and a second (54), wedge-shaped blocking stud facing the connector part's center axis, each blocking stud with one sloping edge, facing the opening (6), and one radially aligned edge, facing away from the opening (6), the second blocking stud (54) being about twice as high as the first (52), the first stud being located nearest the opening, the said blocking studs being arranged to interact with the fixation part during the switch between the contact pin-fixation position and the contact pin-release position, the first blocking studs (52) pressing against and being locked to the fixation part in the contact pin-release position, and the fixation part, in the switch to the contact pin-fixation position, causes the said second blocking studs (54) to detach the fixation part from the first blocking studs.
